# EUROPÄISCHE PATENTSCHRIFT

(11) **EP 2 815 740 B1**
(45) Veröffentlichungstag und Bekanntmachung des Hinweises auf die Patenterteilung: **17.02.2016**
(21) Anmeldenummer: 14172735.4
(22) Anmeldetag: 17.06.2014
(51) Int. Cl.: A61K 9/00, A61K 47/26, A61K 9/08, A61K 47/46, A61K 31/728, A61K 36/80

(54) **Ophtalmische Zusammensetzung**
Ophthalmic composition
Composition ophtalmique

(30) Priorität: 18.06.2013 AT 4892013
(43) Veröffentlichungstag der Anmeldung: 24.12.2014
(73) Patentinhaber: Apomedica pharmazeutische Produkte GmbH, 8010 Graz (AT)
(72) Erfinder: Schmut, Otto, 8020 Graz (AT); Kompek, Albert, 8010 Graz (AT)
(74) Vertreter: KLIMENT & HENHAPEL

(56) Entgegenhaltungen:
- DE-U1-202005 005 094
- JP-A- H1 072 376

## Beschreibung

Die Erfindung betrifft eine ophthalmische Zusammensetzung umfassend Hyaluronsäure oder eines ihrer Metallsalze in physiologisch verträglicher Lösung, gemäß dem Oberbegriff von Anspruch 1.

Hyaluronsäure und ihre Metallsalze finden in ophthalmischen Zusammensetzungen breite Anwendung, etwa zur Behandlung des trockenen Auges. Dabei werden insbesondere ihre vorteilhaften Eigenschaften hinsichtlich ihrer Wasseraufnahmefähigkeit und Viskosität ausgenutzt. Ein Nachteil der Hyaluronsäure besteht in ihrer vergleichsweise schlechten Lagerungsfähigkeit, da sie während der Lagerung oder Anwendung rasch degeneriert. Bei längerer Lagerung oder auch während der Anwendung am Auge nimmt ihre Viskosität zunehmend ab, wodurch sich die Verweildauer des entsprechenden Präparates am Auge verringern. Gründe dafür sind Belastungen durch UV-Strahlung, Ozon oder Feinstaub, die zu einem beschleunigten Abbau der Hyaluronsäure führen. Um die Wirksamkeit der Zusammensetzung zu erhalten, müsste daher ein wiederholtes Applizieren in vergleichsweise kurzen Zeitabständen erfolgen, was sich in der praktischen Anwendbarkeit als mühsam und nachteilig erweist.

Daher wurde vorgeschlagen, durch Zusätze die Viskosität der Zusammensetzung über längeren Zeitraum zu erhalten. So beschreibt etwa die JP 10072376 A ophthalmische Zusammensetzungen enthaltend Hyaluronsäure und/oder deren Salze, wobei Polyole, bevorzugt Ethylenglykol und Glycerin, zur Einstellung der Viskosität verwendet werden, dabei aber höhere Konzentrationen in der Zusammensetzung aufweisen müssen als die Hyaluronsäure als aktive Substanz.

Die EP 1732620 B1 beschreibt viskoelastische Zusammensetzungen für die Anwendung in der ophthalmischen Chirurgie unter anderem enthaltend 0.1-6 w/v-% Hyaluronsäure und/oder deren Salze und 0.1-15 w/v-% eines Polyols (z.B. Mannitol) zur Viskositätsregulierung, wobei aus den Ausführungsbeispielen ersichtlich ist, dass die Menge des beigefügten Polyols jene der Hyaluronsäure um bis zum Vierfachen übersteigen muss.

In der WO 2010/030725 A1 sind ophthalmische Zusammensetzungen speziell für die Behandlung des trockenen Auges beschrieben, die unter anderem 0.001-10 Gewichtsprozent Hyaluronsäure und 0.001-7 Gewichtsprozent eines Polyols (mit Ausnahme von Sorbitol) enthalten. Auch in dieser Druckschrift zeigen die Ausführungsbeispiele, dass die Menge des beigefügten Polyols jene der Hyaluronsäure um bis zum Vierfachen übersteigt, wobei das Polyol gemeinsam mit der Hyaluronsäure der Viskositätseinstellung der Zusammensetzung dient, um die Verweildauer der Zusammensetzung im Auge zu optimieren. Dabei zeigt sich ein synergistischer Effekt von Glycerin, gemeinsam mit Hyaluronsäure die Viskosität der Zusammensetzung zu erhöhen.

Die vergleichsweise hohen Mengen des beigefügten Polyols sowie das synergistische Zusammenwirken bestimmter Polyole mit der Hyaluronsäure erhöhen zwar die Viskosität und somit die Verweildauer der Zusammensetzung im Auge, vermindern jedoch auch die Wirksamkeit der Hyaluronsäure etwa bei der Behandlung des Syndroms des trockenen Auges und können eine Degeneration der Hyaluronsäure während ihrer Anwendung im Auge nicht gänzlich unterbinden. Bekannt ist, dass äußere Einwirkungen wie UV-Strahlung, Ozon-Exposition oder auch Feinstaub einen Abbau der Hyaluronsäure verursachen können, wobei der Wirkmechanismus zumindet zum Teil auf Radikalbildung beruht, im Detail aber noch unverstanden ist. So zeigt sich etwa paradoxer Weise, dass einige Substanzen, die Schutz gegen UV-Stralzlung bieten, sich als wirkungslos gegen Ozon verweisen. Feinstaub ist in Zusammenwirkung mit UV-Strahlung überproportional schädlicher als in Alleinwirkung. Ein weiterer Nachteil hochviskoser Zusammensetzungen besteht ferner darin, dass diese Zusammensetzungen im Auge mitunter als unangenehm empfunden werden, insbesondere während der Anwendung am Tag (Schleiersehen).

Es ist daher das Ziel der Erfindung die Haltbarkeit der Hyaluronsäure in einer ophthalmischen Zusammensetzung während der Lagerung und Anwendung am Auge und somit deren Wirksamkeit auch bei Exposition durch Noxen wie Feinstaub, Ozon oder UV-Strahlung zu verbessern. Dabei soll eine Viskositätserhöhung der Zusammensetzung, die vom Anwender als unangenehm empfunden werden kann, vermieden werden.

Dieses Ziel wird erfindungsgemäß dadurch erreicht, dass der ophthalmischen Zusammensetzung Extrakte erhältlich aus der Pflanze oder aus Pflanzenteilen von Euphrasia officinalis, sowie ein Alditol hinzugefügt werden. Bisherige experimentelle Ergebnisse zeigen, dass der Abbau der Hyaluronsäure durch die Zugabe einer Kombination des Euphrasia-Extraktes und des Alditols zumindest wesentlich verzögert, wenn nicht über mehrere Stunden nach Anwendung sogar annähernd unterbunden werden kann. Vorzugsweise werden die Hyaluronsäure oder eines ihrer Metallsalze in einer Menge von 0.1-1.0 Gewichtsprozent, das Extrakt erhältlich aus der Pflanze oder aus Pflanzenteilen von Euphrasia officinalis in einer Menge von 0.01-10 Gewichtsprozent der Zusammensetzung, und das Alditol in einer Menge von 0.01-1 Gewichtsprozent der Zusammensetzung verwendet. Des Weiteren kann der Zusammensetzung noch Glukose beigemengt werden. Glukose findet sich einerseits auch in natürlicher Tränenflüssigkeit, andererseits deuten Versuche der Anmelderin auch darauf hin, dass Glukose die stabilisierende Wirkung des Alditols und des Euphrasia officinalis-Extraktes unterstützt.

Unter dem Begriff Extrakt werden im Sinne dieser Erfindung Auszüge von flüssiger oder trockener Beschaffenheit von Pflanzen oder deren Teilen, die beispielsweise unter Verwendung von Wasser gewonnen werden, verstanden. Bevorzugt sind Extrakte von trockener Beschaffenheit ("Trockenextrakt"), die vorzugsweise aus den frischen oder getrockneten oberirdischen Teilen von Euphrasia officinalis wie Blätter, Blüten und/oder Stängel erhältlich sind. Unter dem Begriff "Flüssigextrakt" werden im Sinne dieser Erfindung Auszüge von Pflanzen oder deren Teile, die unter Verwendung von Lösemitteln wie Wasser und/oder Alkoholen wie Ethanol hergestellt werden, pflanzliche Presssäfte oder Lösungen von getrockneten Pflanzen in einem Lösemittelgemisch wie einem Alkohol-Wasser-Gemisch verstanden.

Unter der Bezeichnung "Euphrasia officinalis" (Augentrost) werden im Sinne dieser Erfindung alle üblicherweise unter diesem Begriff bezeichneten Arten der Gattung Euphrasia verstanden, beispielsweise Euphrasia stricta, Euphrasia montana und/oder Euphrasia rostkoviana. Die Verwendung von Euphrasia officinalis wurde bereits in mehreren ophthalmischen Zusammensetzungen für unterschiedliche Indikationen beschrieben. Wenngleich im bekannten Stand der Technik Euphrasia officinalis wegen ihrer auch in der Naturheilkunde überlieferten medizinischen Wirkungen verwendet wird, wurde ihre stabilisierende Wirkung auf Hyaluronsäure bislang noch nicht beschrieben. Insbesondere war ihr synergistisches Zusammenwirken mit Alditolen hinsichtlich der Stabilisierung der Hyaluronsäure und ihrer Metallsalze bislang unbekannt. Die Erfindung stellt somit eine Lösung bereit, Hyaluronsäure und ihre Metallsalze auch angesichts komplexer Abbaumechanismen durch äußere Einwirkungen wie UV-Strahlung, Ozon-Exposition oder Feinstaub in einer physiologisch verträglichen Lösung hinlänglich zu stabilisieren. Daher umfasst die Erfindung auch die Verwendung der Kombination eines Alditols und eines Extraktes erhältlich aus der Pflanze oder aus Pflanzenteilen von Euphrasia officinalis zur Stabilisierung einer ophthalmischen Zusammensetzung enthaltend Hyaluronsäure oder eines ihrer Metallsalze in physiologisch verträglicher Lösung. Unter dem Begriff "Stabilisierung" wird hierbei die Beibehaltung oder verzögerte Abnahme einer anfänglichen Viskosität der Zusammensetzung enthaltend Hyaluronsäure oder deren Metallsalze verstanden, sowohl im Ophthalmikum als auch bei der Anwendung am UV-, Ozon- oder Feinstaub-exponierten Auge.

Alditole sind nichtzyklische Polyole mit der Formel HOCH₂[CH(OH)]ₙCH₂OH, die sich strukturell als Reduktionsprodukte von Kohlenhydraten (Zucker) ableiten. Sie werden auch als Zuckeralkohole bezeichnet. Zu den Alditolen zählen beispielsweise Mannit (Mannitol), Isomalt, Lactit, Sorbit (Sorbitol oder Glucitol) und Xylit (Xylitol), Threit, Erythrit und Arabit, oder auch Glycerin (Glycerol). Der Einsatz etwa von Mannitol ist bei ophthalmischen Zusammensetzungen zwar zur Einstellung der Viskosität oder Isotonie bekannt, jedoch nicht zur Stabilisierung von Hyaluronsäure. Dieser Unterschied zeigt sich auch in den vorgeschlagenen Mengen von 0.01-1 Gewichtsprozent des Alditols in der erfindungsgemäßen Zusammensetzung, mit denen eine Einstellung der Isotonie oder der Viskosität nicht wirkungsvoll vorgenommen werden könnte.

Die Beigabe des Alditols erfolgt etwa in derselben Menge wie jene des Extraktes von Euphrasia officinalis. Wie noch gezeigt werden wird, kann somit bei wesentlich geringeren Konzentrationen des Alditols als sie bislang in ophthalmischen Zusammensetzungen verwendet wurden, eine Stabilisierung der Hyaluronsäure erreicht werden, Die erfindungsgemäßen Zusammensetzungen können somit mit Viskositätseigenschaften, die eine angenehmere Anwendung im Auge ermöglichen, ausgestattet werden.

Um den pH-Wert der erfindungsgemäßen ophthalmischen Zusammensetzung einzustellen und im Wesentlichen stabil zu halten (der pH-Wert der Zusammensetzung liegt vorzugsweise zwischen 7 und 7,8, noch mehr bevorzugt zwischen 7,2 und 7,6, insbesondere bei 7,4), ist es erfindungsgemäß bevorzugt einen Hydrogencarbonatpuffer in der erfindungsgemäßen Zusammensetzung vorzusehen. Der erfindungsgemäßen Zusammensetzung wird dabei Hydrogencarbonat in einer Menge beigegeben, die ausreicht, um den pH-Wert der Zusammensetzung zwischen 7 und 7,8, noch mehr bevorzugt zwischen 7,2 und 7,6, insbesondere auf 7,4, einzustellen.

Die Erfindung wird in weiterer Folge anhand der beiliegenden Figuren näher erläutert. Es zeigen hierbei die
Fig. 1 experimentelle Daten zur stabilisierenden Wirkung von Mannit und Euphrasia-Extrakt auf Hyaluronsäure gegenüber einer Ozon-Exposition,
Fig. 2 experimentelle Daten zur stabilisierenden Wirkung von Mannit und Euphrasia-Extrakt auf Hyaluronsäure gegenüber einer UV B-Exposition,
Fig. 3 experimentelle Daten zur stabilisierenden Wirkung von Glycerin und Euphrasia-Extrakt auf Hyaluronsäure gegenüber einer Ozon-Exposition, und die
Fig. 4 experimentelle Daten zur stabilisierenden Wirkung von Glycerin und Euphrasia-Extrakt auf Hyaluronsäure gegenüber einer UV B-Exposition.

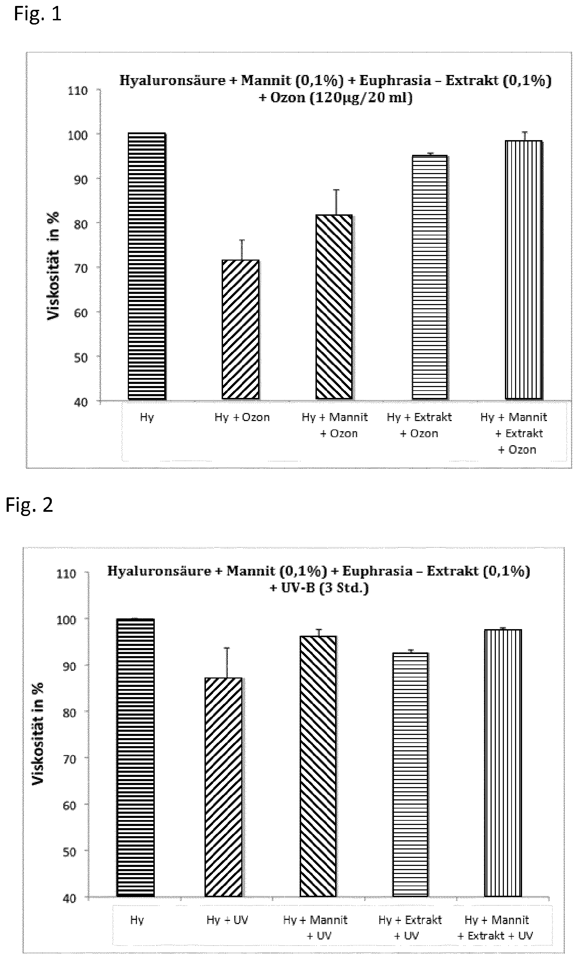

In den Fig. 1-4 wurde die stabilisierende Wirkung einer Kombination eines Extraktes von Euphrasia officinalis mit einem Alditol untersucht. Die Degradation der Hyaluronsäure wurde dabei mittels Ozon-Bedampfung oder mittels Bestrahlung durch UV-B-Licht herbeigeführt. Das Ausmaß der Degradation wurde durch Bestimmung der kinematischen Viskosität der Zusammensetzung ermittelt. Für alle Versuche mit Ozon-Bedampfung wurde die entsprechende Zusammensetzung in Glasfläschchen gefüllt und mit insgesamt 120 µg/20ml Ozon bedampft. Nach der Bedampfung wurde unverzüglich die kinematische Viskosität der Zusammensetzung ermittelt. Für alle Versuche mit UV B-Bestrahlung wurde die entsprechende Zusammensetzung in UV-Licht-durchlässige Quarzglasfläschchen gefüllt und für 3 Stunden auf eine UV B-Lampe gestellt. Nach der Bestrahlung wurde unverzüglich die kinematische Viskosität der Zusammensetzung ermittelt. In den gezeigten Versuchen wurde ein Trockenextrakt von Euphrasia officinalis verwendet, die Wirkung zeigt sich jedoch auch für einen entsprechenden Flüssigextrakt. Die Hyaluronsäure wurde in den gezeigten Messungen in einer Menge von 0.05 Gewichtsprozent in physiologischer Kochsalzlösung verwendet, um die Versuchszeiten zu verkürzen. In den Fig. 1 bis 4 sind die Fehlerbalken lediglich einseitig eingezeichnet, sie erstrecken sich aber symmetrisch zu beiden Seiten des gezeigten Wertes.

In der Fig. 1 sind experimentelle Daten zur stabilisierenden Wirkung von Mannit und Euphrasia-Extrakt auf Hyaluronsäure gegenüber einer Ozon-Exposition zusammen gefasst. Säule 1 zeigt hierbei den Ausgangswert der Viskosität der Hyaluronsäure, die in weiterer Folge mit 100% angenommen wird. Säule 2 zeigt hingegen die Viskosität der Hyaluronsäure nach Bedampfung mit Ozon. Es ist ersichtlich, dass die Viskosität deutlich abgenommen hat, was auf eine Degradation der Hyaluronsäure durch Ozon schließen lässt. Die Säule 3 zeigt im Vergleich dazu die Viskosität einer Mischung enthaltend Hyaluronsäure und 0,1 Gewichtsprozent Mannit nach Bedampfung mit Ozon. Es ist deutlich zu erkennen, dass die Viskosität der Hyaluronsäure weniger stark abgenommen hat wie bei Säule 2. Daraus ist zu schließen, dass die Zugabe von 0,1 Gewichtsprozent Mannit zur Hyaluronsäure einen Schutz vor der Degradation durch Ozon bietet. Die Säule 4 zeigt die Viskosität einer Mischung enthaltend Hyaluronsäure und 0,1 Gewichtsprozent eines Euphrasia officinalis-Trockenextraktes nach Bedampfung mit Ozon. Es ist deutlich zu erkennen, dass die Viskosität der Hyaluronsäure nicht nur im Vergleich zu Säule 2 weniger stark abgenommen hat, sondern auch im Vergleich zu Säule 3. Daraus ist zu schließen, dass die Zugabe von 0,1% Euphrasia officinalis-Trockenextrakt zur Hyaluronsäure einen verbesserten Schutz vor der Degradation durch Ozon bietet. Säule 5 zeigt schließlich die Viskosität einer Zusammensetzung enthaltend Hyaluronsäure, Mannit (0,1 Gewichtsprozent) und Euphrasia officinalis-Trockenextrakt (0,1 Gewichtsprozent) nach Bedampfung mit Ozon. Es ist deutlich zu erkennen, dass die Viskosität der Hyaluronsäure im Vergleich zum Ausgangswert weitestgehend unverändert blieb. Daraus ist zu schließen, dass die Kombination von 0,1% Mannit und 0,1% Euphrasia-Trockenextrakt einen weitestgehend optimalen Schutz vor der Zerstörung von Hyaluronsäure durch Ozon bietet, sowie einen besseren Schutz als eine der beiden Substanzen einzeln eingesetzt.

In der Fig. 2 sind experimentelle Daten zur stabilisierenden Wirkung von Mannit und Euphrasia-Extrakt auf Hyaluronsäure gegenüber einer UV B-Exposition zusammen gefasst. Säule 1 zeigt hierbei den Ausgangswert der Viskosität der Hyaluronsäure, die in weiterer Folge mit 100% angenommen wird. Säule 2 zeigt hingegen die Viskosität der Hyaluronsäure nach Bestrahlung mit UV B-Licht für 3 Stunden. Es ist ersichtlich, dass die Viskosität abgenommen hat, was auf eine Degradation der Hyaluronsäure durch UV B-Licht schließen lässt. Die Säule 3 zeigt im Vergleich dazu die Viskosität einer Mischung enthaltend Hyaluronsäure und 0,1 Gewichtsprozent Mannit nach Bestrahlung mit UV B-Licht für 3 Stunden. Es ist deutlich zu erkennen, dass die Viskosität der Hyaluronsäure weniger stark abgenommen hat wie bei Säule 2. Daraus ist zu schließen, dass die Zugabe von 0,1 Gewichtsprozent Mannit zur Hyaluronsäure einen Schutz vor der Degradation durch UV B-Bestrahlung bietet. Die Säule 4 zeigt die Viskosität einer Mischung enthaltend Hyaluronsäure und 0,1 Gewichtsprozent eines Euphrasia officinalis-Trockenextraktes nach Bestrahlung mit UV B-Licht für 3 Stunden. Es ist deutlich zu erkennen, dass die Viskosität der Hyaluronsäure zwar im Vergleich zu Säule 2 weniger stark abgenommen hat, jedoch stärker als im Vergleich zu Säule 3. Daraus ist zu schließen, dass die Zugabe von 0,1 Gewichtsprozent Mannit zur Hyaluronsäure einen verbesserten Schutz vor der Zerstörung durch UV B-Bestrahlung bietet als die Zugabe von 0,1% Euphrasia officinalis-Trockenextrakt. Säule 5 zeigt schließlich die Viskosität einer Zusammensetzung enthaltend Hyaluronsäure, Mannit (0,1 Gewichtsprozent) und Euphrasia officinalis-Trockenextrakt (0,1 Gewichtsprozent) nach Bestrahlung mit UV B-Licht für 3 Stunden. Es ist deutlich zu erkennen, dass die Viskosität der Hyaluronsäure im Vergleich zum Ausgangswert weitestgehend unverändert blieb. Daraus ist zu schließen, dass die Kombination von 0,1% Mannit und 0,1 % Euphrasia-Trockenextrakt einen weitestgehend optimalen Schutz vor der Zerstörung von Hyaluronsäure durch UV B bietet.

Ein Vergleich der Fig. 1 mit der Fig. 2 zeigt außerdem, dass nicht nur der Wirkmechanismus hinsichtlich der Degradation der Hyaluronsäure durch Ozon-Bedampfung und durch UV B-Bestrahlung unterschiedlich sein dürfte, sondern auch hinsichtlich der stabilisierenden Wirkung von Mannit und Euphrasia officinalis. Eine Kombination von Mannit und Euphrasia officinalis scheint hingegen einen wirksamen Schutz sowohl hinsichtlich Belastungen durch Ozon oder UV B-Licht darzustellen, als auch hinsichtlich einer kombinierten Belastung durch Ozon und UV B-Licht. Eine solche breite Schutzwirkung ist für die Anwendung entsprechender ophthalmischer Zusammensetzungen von großer Bedeutung, da im praktischen Einsatz kombinierte Belastungen oftmals vorliegen, etwa an heißen Tagen durch UV B-Licht und Ozon, oder bei Verwendung der Zusammensetzung in unterschiedlichen Höhenlagen.

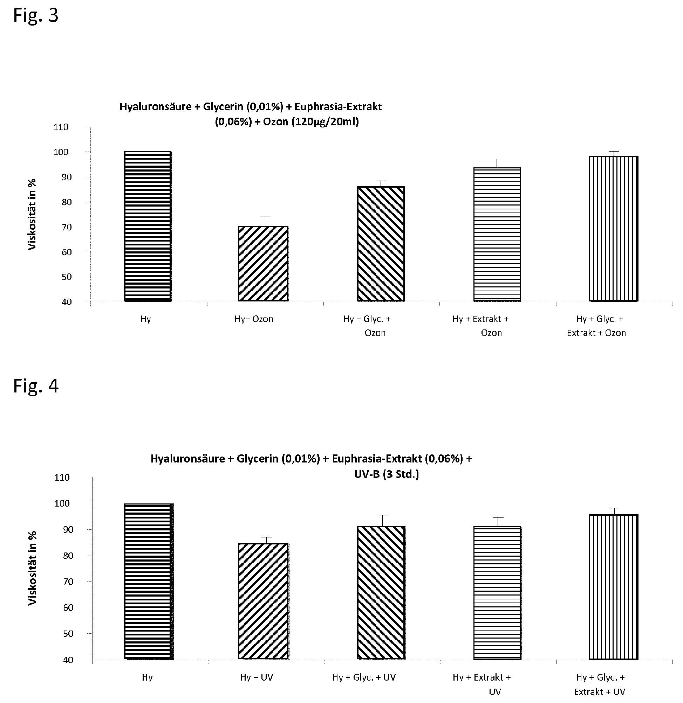

In der Fig. 3 sind experimentelle Daten zur stabilisierenden Wirkung von Glycerin und Euphrasia-Extrakt auf Hyaluronsäure gegenüber einer Ozon-Exposition zusammen gefasst. Säule 1 zeigt hierbei den Ausgangswert der Viskosität der Hyaluronsäure, die in weiterer Folge mit 100% angenommen wird. Säule 2 zeigt hingegen die Viskosität der Hyaluronsäure nach Bedampfung mit Ozon. Es ist ersichtlich, dass die Viskosität deutlich abgenommen hat, was auf eine Degradation der Hyaluronsäure durch Ozon schließen lässt. Die Säule 3 zeigt im Vergleich dazu die Viskosität einer Mischung enthaltend Hyaluronsäure und 0,01 Gewichtsprozent Glycerin nach der Bedampfung mit Ozon. Es ist deutlich zu erkennen, dass die Viskosität der Hyaluronsäure weniger stark abgenommen hat wie bei Säule 2. Daraus ist zu schließen, dass die Zugabe von 0,01 Gewichtsprozent Glycerin zur Hyaluronsäure einen Schutz vor der Degradation durch Ozon bietet. Die Säule 4 zeigt die Viskosität einer Mischung enthaltend Hyaluronsäure und 0,06 Gewichtsprozent eines Euphrasia officinalis-Trockenextraktes nach der Bedampfung mit Ozon. Es ist deutlich zu erkennen, dass die Viskosität der Hyaluronsäure nicht nur im Vergleich zu Säule 2 weniger stark abgenommen hat, sondern auch im Vergleich zu Säule 3. Daraus ist zu schließen, dass die Zugabe von 0,06% Euphrasia officinalis-Trockenextrakt zur Hyaluronsäure einen verbesserten Schutz vor der Zerstörung durch Ozon bietet. Säule 5 zeigt schließlich die Viskosität einer Zusammensetzung enthaltend Hyaluronsäure, Glycerin (0,01 Gewichtsprozent) und Euphrasia officinalis-Trockenextrakt (0,06 Gewichtsprozent) nach Bedampfung mit Ozon. Es ist deutlich zu erkennen, dass die Viskosität der Hyaluronsäure im Vergleich zum Ausgangswert weitestgehend unverändert blieb. Daraus ist zu schließen, dass die Kombination von 0,01% Glycerin und 0,06% Euphrasia-Trockenextrakt einen weitestgehend optimalen Schutz vor der Zerstörung von Hyaluronsäure durch Ozon bietet, sowie einen besseren Schutz als eine der beiden Substanzen einzeln eingesetzt.

In der Fig. 4 sind experimentelle Daten zur stabilisierenden Wirkung von Glycerin und Euphrasia-Extrakt auf Hyaluronsäure gegenüber einer UV B-Exposition zusammen gefasst. Säule 1 zeigt hierbei den Ausgangswert der Viskosität der Hyaluronsäure, die in weiterer Folge mit 100% angenommen wird. Säule 2 zeigt hingegen die Viskosität der Hyaluronsäure nach Bestrahlung mit UV B-Licht für 3 Stunden. Es ist ersichtlich, dass die Viskosität abgenommen hat, was auf eine Degradation der Hyaluronsäure durch UV B-Licht schließen lässt. Die Säule 3 zeigt im Vergleich dazu die Viskosität einer Mischung enthaltend Hyaluronsäure und 0,01 Gewichtsprozent Glycerin nach Bestrahlung mit UV B-Licht für 3 Stunden. Es ist deutlich zu erkennen, dass die Viskosität der Hyaluronsäure weniger stark abgenommen hat wie bei Säule 2. Daraus ist zu schließen, dass die Zugabe von 0,01 Gewichtsprozent Glycerin zur Hyaluronsäure einen Schutz vor der Degradation durch UV B-Bestrahlung bietet. Die Säule 4 zeigt die Viskosität einer Mischung enthaltend Hyaluronsäure und 0,06 Gewichtsprozent eines Euphrasia officinalis-Trockenextraktes nach Bestrahlung mit UV B-Licht für 3 Stunden. Es ist zu erkennen, dass die Viskosität der Hyaluronsäure zwar im Vergleich zu Säule 2 weniger stark abgenommen hat, jedoch etwas stärker als im Vergleich zu Säule 3. Daraus ist zu schließen, dass die Zugabe von 0,01 Gewichtsprozent Glycerin zur Hyaluronsäure einen tendenziell besseren Schutz vor der Zerstörung durch UV B-Bestrahlung bietet als die Zugabe von 0,06% Euphrasia officinalis-Trockenextrakt. Säule 5 zeigt schließlich die Viskosität einer Zusammensetzung enthaltend Hyaluronsäure, Glycerin (0,01 Gewichtsprozent) und Euphrasia officinalis-Trockenextrakt (0,06 Gewichtsprozent) nach Bestrahlung mit UV B-Licht für 3 Stunden. Es ist zu erkennen, dass die Viskosität der Hyaluronsäure im Vergleich zum Ausgangswert weitestgehend unverändert blieb. Daraus ist zu schließen, dass die Kombination von 0,01% Glycerin und 0,06% Euphrasia-Trockenextrakt einen weitestgehend optimalen Schutz vor der Zerstörung von Hyaluronsäure durch UV B bietet.

Es wird angemerkt, dass eine Vielzahl an Versuchen für weitere Substanzen keine Schutzwirkung gegenüber einer Degradation der Hyaluronsäure gezeigt haben, sondern im Gegenteil die Degradation der Hyaluronsäure mitunter sogar begünstigt haben. Diese Versuche umfassten etwa Melatonin, Harnsäure, Taurin, Spermidin, Lysin, Arginin, Curcumin oder Harnstoff. Diese Substanzen werden im Stand der Technik für verschiedene Zwecke als Zusätze für ophthalmische Zusammensetzungen genannt und verleiten zur Annahme, dass eine Stabilisierung der Hyaluronsäure insbesondere gegenüber kombinierten Belastungen nicht möglich wäre. Hinsichtlich Glukose und Fruktose existieren hingegen Hinweise auf eine unterstützende Wirkung zur Stabilisierung der Hyaluronsäure.

Für den praktischen Einsatz wird eine ophthalmische Zusammensetzung enthaltend Hyaluronsäure oder eines ihrer Metallsalze in physiologisch verträglicher Lösung, ein Alditol, sowie einen Extrakt erhältlich aus der Pflanze oder aus Pflanzenteilen von Euphrasia officinalis vorgeschlagen. Eine mögliche Zusammensetzung enthält etwa Hyaluronsäure oder eines ihrer Metallsalze in einer Menge von 0,25 Gewichtsprozent, das Extrakt erhältlich aus der Pflanze oder aus Pflanzenteilen von Euphrasia officinalis in einer Menge von 0,1 Gewichtsprozent der Zusammensetzung, und das Alditol in einer Menge von 0,1 Gewichtsprozent der Zusammensetzung. Diese Zusammensetzung kann, falls erforderlich, mit allfällig weiteren Zusätzen versehen sein. Im Rahmen der Erfindung erscheint insbesondere die Beimengung von Glukose, etwa in einer Menge von 0,005 Gewichtsprozent, als vorteilhaft, da sie die stabilisierende Wirkung des Alditols und des Euphrasia officinalis-Extraktes auf die Hyaluronsäure zu unterstützen scheint. Zur Isotonisierung kann Natriumchlorid verwendet werden. Um den pH-Wert der erfindungsgemäßen ophthalmischen Zusammensetzung einzustellen und im Wesentlichen stabil zu halten (der pH-Wert der Zusammensetzung liegt vorzugsweise zwischen 7 und 7,8, noch mehr bevorzugt zwischen 7,2 und 7,6, insbesondere bei 7,4), ist es erfindungsgemäß bevorzugt einen Hydrogencarbonatpuffer in der erfindungsgemäßen Zusammensetzung vorzusehen. Der erfindungsgemäßen Zusammensetzung wird dabei Hydrogencarbonat in einer Menge beigegeben, die ausreicht, um den pH-Wert der Zusammensetzung zwischen 7 und 7,8, noch mehr bevorzugt zwischen 7,2 und 7,6, insbesondere auf 7,4, einzustellen.

Die Zusammensetzung wird dabei etwa als Augentropfen in entsprechenden Behältnissen bereitgestellt, die vorzugsweise so gestaltet sind, dass der Inhalt auf das Auge aufgebracht werden kann.

Die Erfindung stellt somit eine Lösung bereit, Hyaluronsäure und ihre Metallsalze auch angesichts komplexer Abbaumechanismen durch äußere Einwirkungen wie UV-Strahlung, Ozon-Exposition oder Feinstaub in einer physiologisch verträglichen Lösung hinlänglich zu stabilisieren. Des Weiteren kann auch mit wesentlich geringeren Konzentrationen des Alditols als sie bislang in ophthalmischen Zusammensetzungen verwendet wurden, eine Stabilisierung der Hyaluronsäure erreicht werden. Die erfindungsgemäßen Zusammensetzungen können somit mit Viskositätseigenschaften, die eine angenehmere Anwendung im Auge ermöglichen, ausgestattet werden.

## Patentansprüche

1. Ophthalmische Zusammensetzung umfassend Hyaluronsäure oder eines ihrer Metallsalze in physiologisch verträglicher Lösung, **dadurch gekennzeichnet, dass** sie Extrakte erhältlich aus der Pflanze oder aus Pflanzenteilen von Euphrasia officinalis, sowie ein Alditol umfasst.

2. Ophthalmische Zusammensetzung nach Anspruch 1, **dadurch gekennzeichnet, dass** die Hyaluronsäure oder eines ihrer Metallsalze in einer Menge von 0,1-1,0 Gewichtsprozent, das Extrakt erhältlich aus der Pflanze oder aus Pflanzenteilen von Euphrasia officinalis in einer Menge von 0,01-10 Gewichtsprozent der Zusammensetzung, und das Alditol in einer Menge von 0,01-1 Gewichtsprozent der Zusammensetzung vorliegt.

3. Ophthalmische Zusammensetzung nach Anspruch 1 oder 2, **dadurch gekennzeichnet, dass** zusätzlich Glukose enthalten ist.

4. Ophthalmische Zusammensetzung nach einem der Ansprüche 1 bis 3, **dadurch gekennzeichnet, dass** die Zusammensetzung einen Hydrogencarbonatpuffer umfasst.

5. Verwendung der Kombination eines Alditols und eines Extraktes erhältlich aus der Pflanze oder aus Pflanzenteilen von Euphrasia officinalis zur Stabilisierung einer ophthalmischen Zusammensetzung enthaltend Hyaluronsäure oder eines ihrer Metallsalze in physiologisch verträglicher Lösung.

## Claims

1. An ophthalmic composition, comprising hyaluronic acid or one of its metal salts in physiologically compatible solution, **characterized in that** it comprises extracts obtainable from the plant or plant parts of euphrasia officinalis, as well as an alditol.

2. An ophthalmic composition according to claim 1, **characterized in that** the hyaluronic acid or one of its metal salts is present in a quantity of 0.1 to 1.0 percent by weight, the extract obtainable from the plant or plant parts of euphrasia officinalis in a quantity of 0.01 to 10 percent by weight of the composition, and the alditol in a quantity of 0.01 to 1 percent by weight of the composition.

3. An ophthalmic composition according to claim 1 or 2, **characterized in that** glucose is additionally contained.

4. An ophthalmic composition according to one of the claims 1 to 3, **characterized in that** the composition comprises a hydrogen carbonate buffer.

5. The use of a combination of an alditol and an extract obtainable from the plant or plant parts of euphrasia officinalis for stabilising an ophthalmic composition containing hyaluronic acid or one of its metal salts in physiologically compatible solution.

## Revendications

1. Composition ophtalmique contenant de l'acide hyaluronique ou l'un de ses sels métalliques en solution physiologiquement compatible, **caractérisée en ce qu'**elle contient des extraits obtenus à partir de la plante ou de parties de la plante d'*Euphrasia officinalis,* et contient un alditol.

2. Composition ophtalmique selon la revendication 1, **caractérisée en ce que** l'acide hyaluronique ou l'un de ses sels métalliques est présent dans une quantité de 0,1 à 1,0 % en poids, l'extrait obtenu à partir de la plante ou de parties de la plante d'*Euphrasia officinalis* dans une quantité de 0,01 à 10 % en poids de la composition et l'alditol dans une quantité de 0,01 à 1 % en poids de la composition.

3. Composition ophtalmique selon la revendication 1 ou 2, **caractérisée en ce qu'**elle contient en outre du glucose.

4. Composition ophtalmique selon l'une des revendications 1 à 3, **caractérisée en ce que** la composition contient un tampon hydrogénocarbonate.

5. Utilisation de la combinaison d'un alditol et d'un extrait obtenu à partir de la plante ou de parties de la plante d'*Euphrasia officinalis* pour la stabilisation d'une composition contenant de l'acide hyaluronique ou l'un de ses sels métalliques en solution physiologiquement compatible.
